# EUROPEAN PATENT SPECIFICATION

(11) **EP 4 552 682 B1**
(45) Date of publication and mention of the grant of the patent: **28.01.2026**
(21) Application number: 24211397.5
(22) Date of filing: 07.11.2024
(51) Int. Cl.: A61M 27/00, A61B 17/3209, A61B 17/00, A61F 2/04, A61F 2/82, A61F 2/962

(54) **DRUG-DISPENSING URETHRAL IMPLANT**
URETHRALES IMPLANTAT ZUR ABGABE VON ARZNEIMITTELN
IMPLANT URÉTRAL DISTRIBUANT UN MÉDICAMENT

(30) Priority: 12.11.2023 US 202363548191 P
(43) Date of publication of application: 14.05.2025
(73) Proprietor: Medi-Tate Ltd., 3850169 Hadera (IL)
(72) Inventor: Kilemnik, Ido, 4286500 HANIEL (IL)
(74) Representative: Hoener, Matthias

(56) References cited:
- EP-B1- 3 089 780
- CN-A- 113 855 320
- CN-U- 210 811 781
- US-A1- 2021 022 594

## Description

### FIELD OF THE DISCLOSED TECHNIQUE

The disclosed technique relates to medical implant devices, in general, and to drug-dispensing urethral implant devices, in particular.

### BACKGROUND OF THE DISCLOSED TECHNIQUE

Urethral implants in general are known in the art. There is a category of urethral implants that are implanted within the urethras of men, employed for treating benign prostatic hyperplasia (BPH). BPH is a medical condition, also known as prostate enlargement in which the prostate gland increases in size leading to a variety of medical issues such as urination problems (e.g., weak urine drainage, bladder control loss, slow or prolonged urination, increased urination frequency), as well as more serious conditions that can lead to lower urinary tract infections (LUTS), bladder stones, kidney and/or bladder damage, etc. Various treatment options have been proposed and utilized over the years, which include oral medications (e.g., alpha blockers), invasive surgical procedures (e.g., transurethral resection of the prostate (TURP), transurethral incision of the prostate (TUIP)), as well as minimally invasive treatments (e.g., prostatic stent insertion, transurethral microwave thermotherapy (TUMT), transurethral needle ablation (TUNA), transurethral electrovaporization (TUVP)), Holmium laser enucleation of the prostate (HoLEP), etc. Each therapeutic technique has its inherent advantages and disadvantages.

One category of such techniques employs the use of a urethral implant device, such as that disclosed in U.S. Patent No.: US 9,848,905 B2, titled "Radial Cutter Implant" to Kilemnik, which is directed at a method for extending a urinal passage and an implant for creating incisions in the tissues surrounding the bladder neck and the urethra of a patient. According to one embodiment, the implant includes four wires, an anchoring leaflet, a distal end, and a proximal end. Each of the wires is coupled between the proximal end and the distal end. The anchoring leaflet extends from the distal end and is positioned between two of the wires. The wires are wider at the proximal end than at the distal end to prevent the implant from moving from the bladder neck into the urethra wen implanted. The anchoring leaflet prevents the implant from moving from the bladder neck into the bladder.

The method involves inducing infarction via the application of continuous radial pressure an at least one of urethral wall and tissue surrounding the urethral wall. The method includes the procedures of delving the implant (that is removable) to a vicinity of at least one of a bladder neck of a bladder and a prostatic urethra, releasing the removable implant, and applying continuous pressure by the removable implant. The removable implant has at least one foldable wire and an anchor capable of seif-expanding. Once the implant is released, both the foldable wire and anchor expand to prevent movement of the implant along the urethral wall in a direction toward the bladder. The applied continuous pressure creates longitudinal incisions in the urethral wall and in tissue surrounding the urethral wall in a radial direction from a urethral axis outwardly. At an appearance of a predetermined condition, the removable implant is extracted.

U.S. Patent No.: US 11,304,724 B2, titled "Incising Implant for the Prostatic Urethra" to Kilemnik, is directed at an incising implant for creating incisions in the prostatic urethra of a subject. The implant includes at least two closed-shaped wires, and an anchor. Each wire has a proximal section, a distal section and two longitudinal sections that extend between the proximal section and the distal section. Each of the closed-shaped wires is elastic thereby being compressible from an expanded configuration to a compressed configuration. The longitudinal sections of each of the wires are adjoined with another longitudinal section of another one of the wires along their length. In the expanded configuration the incising implant is configured to incise tissue in the prostatic urethra. The at least two wires diverge at their distal sections such that the distal sections form a simple closed curve shape at a terminus of the incising implant in the expanded configuration. The anchor is closed-shaped and extends outwardly from the proximal section and though one of the closed-shaped wires in the expanded configuration.

Other incising implants for creating incisions in the prostatic urethra of a subject are disclosed in CN 113 855 320 A and US 2021/022594 A1, whereby in the document US 2021/022594 A1 it is also disclosed that a drug release could take place by the implant.

CN 210 811 781 U discloses an expandable stent which can be coated with drugs.

### SUMMARY OF THE PRESENT DISCLOSED TECHNIQUE

The invention is defined in the claims.

It is an object of the disclosed technique to provide a novel implant device for the urethra of a subject. The implant includes at least two struts, an anchor, and at least one drug dispensing wire. The at least two struts extend longitudinally between a proximal end and a distal end of the implant. Each of the struts is flexible between a compressed configuration and an expanded configuration. The compressed configuration enables delivery of the implant to a stricture in the urethra. The expanded configuration facilitates expansion of the stricture. The anchor extends outwardly from at least one of the proximal end and the distal end. The anchor is for anchoring the implant in the urethra. The at least one drug dispensing wire is adjoined to and extends longitudinally along, at least one of the struts. The drug dispensing wire is embedded with a drug for releasing into the urethra. In accordance with the disclosed technique, there is thus provided an implant for the urethra of a subject. The implant includes three closed-shaped wires, an anchor, and at least one drug dispensing wire. Each closed-shape wire includes two longitudinal sections extending between a proximal end and a distal end of the implant. Each longitudinal section of a closed-shaped wire is adjoined along its length with another longitudinal section of another closed-shaped wire. The closed-shaped wires are configured to engage with and to expand urethral tissue at a stricture in the urethra. The anchor includes a closed-shape wire. The anchor is for anchoring the implant to the urethra. The at least one drug dispensing wire is, adjoined to and extends longitudinally along at least one of the the struts. The drug dispensing wire is embedded with a drug for releasing into the urethra. In accordance with the disclosed technique, there is thus provided an implant for the urethra of a subject. The implant includes three closed-shaped wires, an anchor, and at least one drug dispensing wire. Each closed-shape wire includes two longitudinal sections extending between a proximal end and a distal end of the implant. Each longitudinal section of a closed-shaped wire is adjoined along its length with another longitudinal section of another closed-shaped wire. The closed-shaped wires are configured to engage with and to expand urethral tissue at a stricture in the urethra. The anchor includes a closed-shape wire. The anchor is for anchoring the implant to the urethra. The at least one drug dispensing wire is, adjoined to and extends longitudinally along at least one of the closed-shaped wires. The drug dispensing wire is embedded with a drug for releasing to the urethra.

### BRIEF DESCRIPTION OF THE DRAWINGS

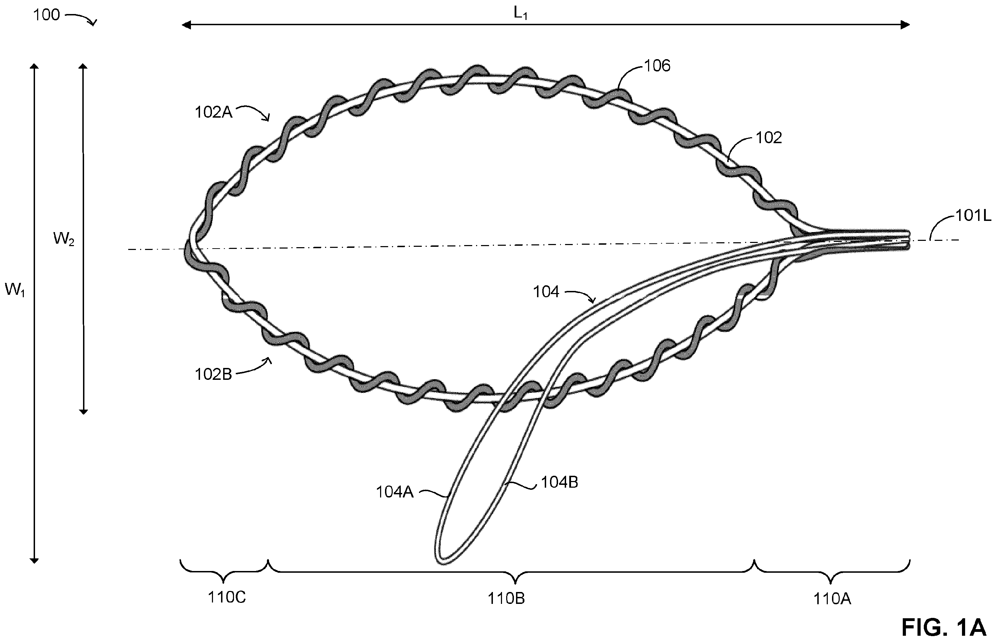

The disclosed technique will be understood and appreciated more fully from the following detailed description taken in conjunction with the drawings in which: Figure 1A is a schematic illustration of a urethral implant, shown in an expanded configuration, constructed and operative in accordance with an embodiment of the disclosed technique;
Figure 1B is a schematic illustration of the urethral implant of Figure 1A including a proximal cap, constructed and operative in accordance with the embodiment of the disclosed technique;
Figure 1C is the urethral implant of Figure 1B in a compressed configuration, constructed and operative in accordance with the embodiment of the disclosed technique;
Figure 2 is a schematic illustration of the urethral implant, shown in an expanded configuration, having twisted wires, constructed and operative in accordance with another embodiment of the disclosed technique;
Figure 3A is a schematic illustration of a urethral implant, shown in an expanded configuration, constructed and operative in accordance with a further embodiment of the disclosed technique;
Figure 3B is a schematic illustration of the urethral implant of Figure 3A including a proximal cap, constructed and operative in accordance with the embodiment of the disclosed technique;
Figure 3C is a schematic illustration of the urethral implant of Figure 3B shown from a distal-to-proximal direction A, constructed and operative in accordance with the embodiment of the disclosed technique;
Figure 4 is a schematic illustration of a urethral implant having a plurality of discontinuous drug dispensing wires, constructed and operative in accordance with another embodiment of the disclosed technique;
Figure 5 is a schematic illustration of a urethral implant having drug dispensing wires coupled to closed-shaped wires that include an anchor, constructed and operative in accordance with a further embodiment of the disclosed technique; and
Figures 6A, 6B, 6C, and 6D, are schematic illustrations of various configurations of drug dispensing wires, constructed and operative in accordance with the embodiments of the disclosed technique.

### DETAILED DESCRIPTION OF THE EMBODIMENTS

The disclosed technique overcomes the disadvantages of the prior art by providing an implant for the urethra (herein "urethral implant") of a subject (i.e., patient, person, implantee), in which the implant includes at least two struts, an anchor, and at least one drug dispensing wire. The at least two struts can be a single wire structure (e.g., a closed-shape loop). The implant includes a proximal end, a distal end, and a central-portion (middle-portion) disposed between the proximal and distal ends. The struts extend longitudinally between the proximal end and the distal end of the implant. The struts are flexible between a compressed configuration that enables delivery of the implant to a stricture (i.e., a narrowing, constriction) in the urethra, and an expanded configuration that facilitates expansion of the urethral passage in general, and the stricture in particular. The anchor extends outwardly from at least one of the proximal end and the distal end, and is configured and operative for anchoring the implant within the urethra of the subject. The drug dispensing wire is adjoined to, and extends longitudinally along, at least one of the struts. The drug dispensing wire is embedded with a drug for releasing into the urethra of the subject. The at least two struts are made of a single continuous wire that forms a closed-shape. Alternatively, the at least two struts are separate entities joined together to form a continuous wire.

The struts generally provide a mechanical structural framework to the urethral implant that allows it to compress and to expand. The drug dispensing wire generally provides a therapeutic action of the drug to target tissues of the urethra (e.g., stricture). The urethral implants of the disclosed technique provide a synergistic effect between the mechanical actions of the wire (struts) and the therapeutic action of drug dispensing wire that extends along a wire-to-tissue engaging area (i.e., the area of which the wire (struts) contact tissues of the subject within the urethra). Specifically, the wires (struts) mechanically engage and apply pressure to urethral tissues at the wire-to-tissue engaging area, while simultaneously the drug dispensing wire makes contact with the same urethral tissues as wire (struts) and delivers a drug to those tissues (i.e., targeted drug delivery).

The urethral implant of the disclosed technique is configured and operative to self-expand by employing shape-memory alloy (SMA) materials, such as nickel titanium (NiTi) (also known by the trade name "Nitinol" (Nickel Titanium Naval Ordinance Laboratories)), having a memorized expanded and compressed configurations. The urethral implant structure is generally hollow to allow urine to pass therethrough (i.e., through the voids) when the urethral implant is implanted in the expanded configuration within the urethra of the subject. The urethral implants of the disclosed technique are capable of incising tissues, specifically, the wires are configured to apply continuous (ischemic) pressure on tissues of the urethra (e.g., prostatic urethra) thereby creating incisions (i.e., longitudinal struts or wires creating corresponding longitudinal incisions), which can lead to infarction or tissue necrosis thus reshaping and expanding the urethra so urine can flow more easily for subjects suffering from BPH). In addition, the wires can be equipped with at least one jagged edge (e.g., along the length of the wires or at least partially along the length thereof) for further facilitating the creation of incisions. In an alternative configuration where the urethral implant doesn't incise tissues of the urethra, it doesn't include jagged edges.

The urethral implants are generally removable from the subject (via an extraction mechanism). It is noted that all urethral implants disclosed herein are non-vascular implants, i.e., not implanted within blood vessels where blood flows, but rather implanted within the urethra where urine flows.

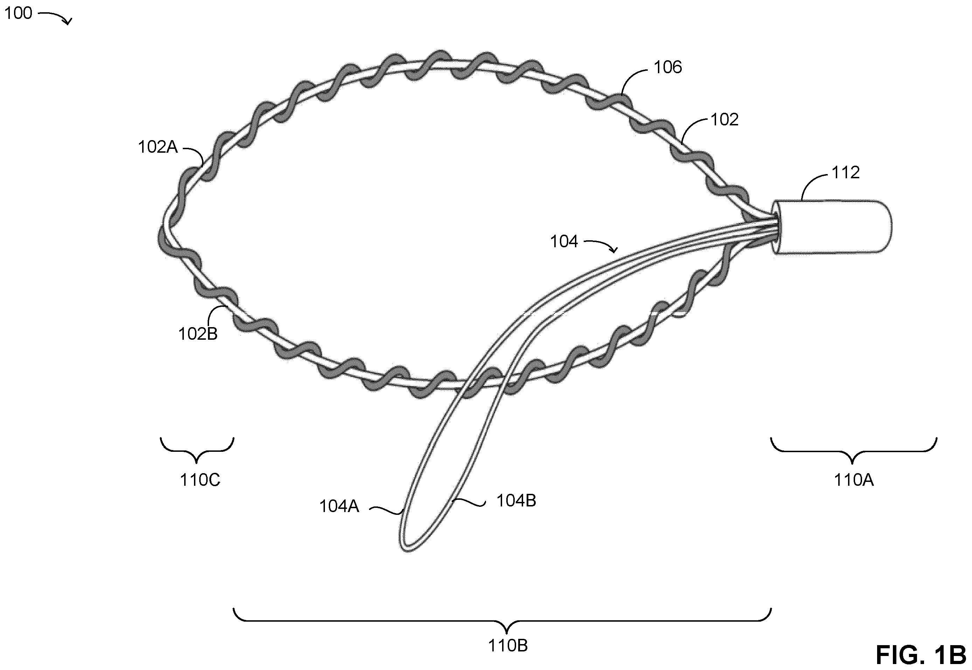

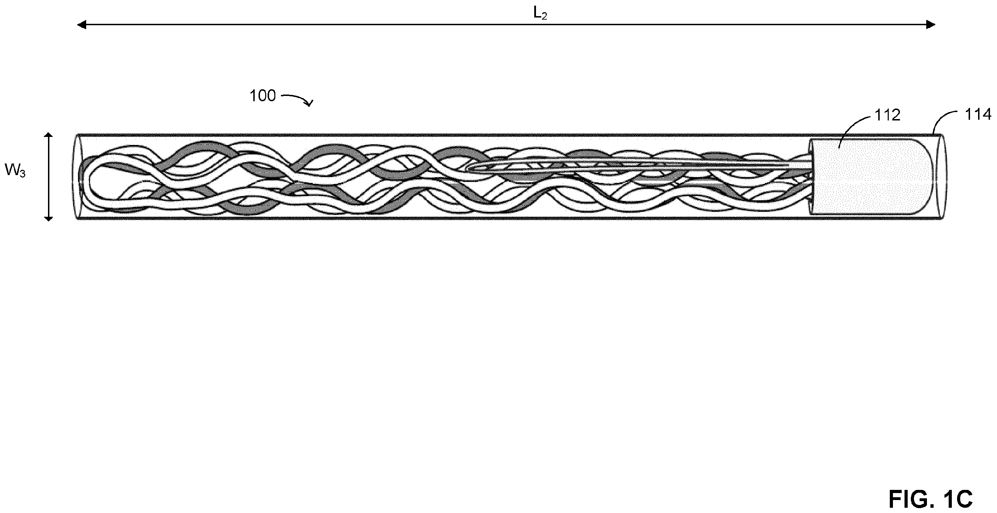

Reference is now made to Figures 1A, 1B, and 1C. Figure 1A is a schematic illustration of a urethral implant, generally referenced 100, shown in an expanded configuration, constructed and operative in accordance with an embodiment of the disclosed technique. Figure 1B is a schematic illustration of the urethral implant of Figure 1A including a proximal cap, constructed and operative in accordance with the embodiment of the disclosed technique. Figure 1C is the urethral implant of Figure 1B in a compressed configuration, constructed and operative in accordance with the embodiment of the disclosed technique. Urethral implant 100 includes a tissue engaging wire 102 (interchangeably denoted herein as "wire"), a tissue engaging anchor 104 (interchangeably denoted herein as "anchor"), and a drug dispensing wire 106.

Wire 102 includes two struts 102A and 102B extending longitudinally along a proximal portion 110A (interchangeably denoted herein "proximal end"), a central portion 110B (interchangeably denoted herein "mid-section"), and a distal portion 110C (interchangeably denoted herein "distal end"), and generally along a longitudinal axis 101L of urethral implant 100. Two struts 102A and 102B shown in Figures 1A and 1B are continuous, i.e., are made of a single piece of wire, which converge at proximal end 110A. Alternatively, struts 102A and 102B are separate pieces (not shown) that are mechanically joined together at distal end 110C, and converging at proximal end 110A. Central portion 110B is defined to be between proximal end 110A and distal end 110C. The length of central portion 110B along longitudinal axis 101L is generally longer than the combined lengths of proximal portion 110A and distal portion 110C. Urethral implant 100 is compressible between an expanded configuration having a length L₁ and a width W₁ (strut width W₂), and a compressed configuration having a length L₂ and a width W₃ as shown in Figure 1C (strut width W₄ - not shown). Figures 1A and 1B show that struts 102A and 102B of wire 102 are geometrically symmetric about longitudinal axis 101L (having the same geometric properties such as equal length, width, and curvature), as well as materially the same (e.g., made of the same material and having the same mechanical properties such as elasticity, strength, malleability, resilience, and the like). A viable alternative configuration (not shown) is where struts 102A and 102B are geometrically asymmetric about longitudinal axis 101L (e.g., at least one of their lengths, widths, curvatures, or other geometrical properties are different with respect to each other), or materially different (i.e., the struts are not made of exactly the same material or have different mechanical properties such as flexibility, strength, and the like). Wire 102 is made of medical-grade bio-compatible materials such as titanium, gold, stainless steel, metal alloys such as nickel titanium (Nitinol), tantalum alloy, and the like. According to an alternative configuration, wire 102 is made of a polymer (e.g., a shape-memory polymer).

Figures 1A, 1B, and 1C show urethral implant 100 in an expanded configuration. When urethral implant 100 is implanted in the subject, it facilitates expansion of a stricture within the urethra of the subject, by applying pressure to surrounding tissues. In this respect, urethra implant 100 generally functions as a mechanical spring that is compressible and expandable having intermediate compression states between two extremities (i.e., fully expanded configuration and fully compressed configuration). The main structural members of urethral implant, namely, struts 102A and 102B are flexible between the compressed configuration (shown in Fig. 1C) that enables delivery of urethral implant 100 to the stricture in the urethra, and the expanded configuration (shown in Figs. 1A and 1B). With specific reference to Figure 1C, a sheath 114 is configured and operative to enfold urethral implant 100 in the compressed configuration, which allows delivery of urethral implant 100 within the body of the subject via a delivery device (not shown) toward a desired anatomical location within the urethra, typically to the stricture, where urethral implant is deployed by releasing from sheath 114 thereby self-expanding to the expanded configuration shown in Figures 1A and 1B. Sheath 114 is typically a hollow tube (e.g., cylindrical and of medical-grade) that is configured to cover, encapsulate, enfold and compress urethral implant 100 in the compressed configuration.

Anchor 104 is typically a continuous wire structure that includes two sides embodied as two struts 104A and 104B, which extend outwardly from proximal end 110A into central portion 110B, to one side of urethral implant 100 of longitudinal axis 101L (i.e., a proximal-to-distal direction). Struts 104A and 104B of anchor 104 are generally made from a single continuous piece of wire converging at proximal end 110A. Alternatively, struts 104A and 104B are separate pieces (not shown) that are mechanically joined together at an apex (i.e., a tip of anchor 104). Anchor 104 is flexible between the expanded configuration (Figures 1A and 1B) and the compressed configuration (Figure 1C). Anchor 104 has the capability to self-expand into the expanded configuration once released from sheath 114. Anchor 104 is generally arcuate defining a closed-shape (e.g., closed loop) having an internal hole and bilateral symmetry of the two struts. Alternatively, anchor 104 is bilaterally asymmetric. Anchor 104 is constructed and operative to engage with, and attach (anchor) to tissues of the urethra of the subject in the expanded configuration. Anchor 104 can be detached from tissues when urethral implant 100 is removed from the subject. Anchor 104 is made of the same material of wire 102. Alternatively, anchor 104 is not made of exactly the same materials of wire 102. Typically, the wire making up anchor 104 is thinner than the wire making up wire 102, as shown in Figures 1A and 1B. Alternatively, the wires making up anchor 104 and wire 102 have the same thickness (not shown). Other alternative configurations of anchor 104 are possible (not shown) (e.g., a closed polygonal shape, etc.).

The breadth of expansion of urethral implant 100 in the urethra is subject-specific (i.e., depending on anatomical factors, as well stricture-dependent factors (e.g., stricture dimensions, stricture tissue characteristics, etc.). According to an alternative embodiment, there is a plurality of anchors (not shown), such as a second anchor that extends outwardly from distal end 110C into central portion 110B at the other side of urethral implant 100 with respect to longitudinal axis 101L (i.e., the side that is different from the extension of the first anchor). Further alternatively, there are two anchors (not shown) constructed and operative to extend from the same end (i.e., either proximal end 110A or distal end 110C) toward its contrariwise direction (i.e., either a distal-to-proximal direction defined from distal end 110C to proximal end 110A, or a proximal-to-distal direction defined from proximal end 110A to distal end 110C).

Drug dispensing wire 106 is adjoined (coupled) to (e.g., continuously wound around) wire 102 along the length of struts 102A and 102B starting and ending at proximal end 110A (i.e., the two ends of drug dispensing wire 106 end at proximal end 110A). The two ends of drug dispensing wire 106 can either be open or closed. The two ends of drug dispensing wire 106 make contact with each other thus defining a closed shape. Drug dispensing wire 106 is adjoined to struts 102A and 102B by helically winding along the entire length thereof (as shown in Figures 1A and 1B). An alternative coupling configuration between drug dispensing wire 106 and closed-shaped wire 102 is parallel coupling (both wires extending in parallel to each other, i.e., non-helically - not shown). Drug dispensing wire 106 has an overall length that is greater than the length of wire 102. Drug dispensing wire 106 is embedded with a drug that is dispensed (i.e., released, administered) into the urethra when urethral implant 100 is implanted within the subject. The drug includes a chemical substance that is configured to produce or perform a biological effect on the subject. Example drugs (medications) include sirolimus (rapamycin), and everolimus. The drugs may generally be formulated to include a time-release mechanism (e.g., liposomes) also known as modified-release dosage that functions to deliver the drug over a period of time (i.e., gradually, and not "immediate-release" formulations) that dissolves, diffuses (or both) at a controlled rate (e.g., controlled release, sustained release, delayed-release, extended release, etc.). The drug can be coated (fully or partially) onto drug dispensing wire 106.

With particular reference to Figures 1B and 1C, proximal end 110A of urethral implant 100 includes a proximal cap 112 that covers the ends (termini) of the wire 102, the ends of the wire of anchor 104, as well the ends of drug dispensing wire 106. Proximal cap 112 is typically made of a medical-grade plastic material (e.g., polyethylene that substantially doesn't degrade within the body, etc.).

Prior to drug dissolution in the body following implantation, drug dispensing wire 106 has a greater surface contact area (i.e., implant-to-tissue engaging surface area) in comparison to that of wire 102. Once drug dispensing wire 106 is implanted, the drug is configured to gradually dissolve at a controlled rate according to its characteristic time-release drug profile. Drug dispensing wire 106 is made of a single strand or multiple strands of a material (e.g., fibrous such as cotton fiber, synthetic fiber such as polyester, etc.) that can absorb as well as exude the embedded drug when urethral implant 100 is implanted. In addition or alternatively, drug dispensing wire 106 is made of a biodegradable material that dissolves when implanted in the urethra of the implantee. According to this alternative, both the embedded drug and drug dispensing wire 106 dissolve over time (e.g., at the same rate, or at different rates). Drug dispensing wire 106 is compressible between the compressed configuration and expanded configuration of urethral implant 100.

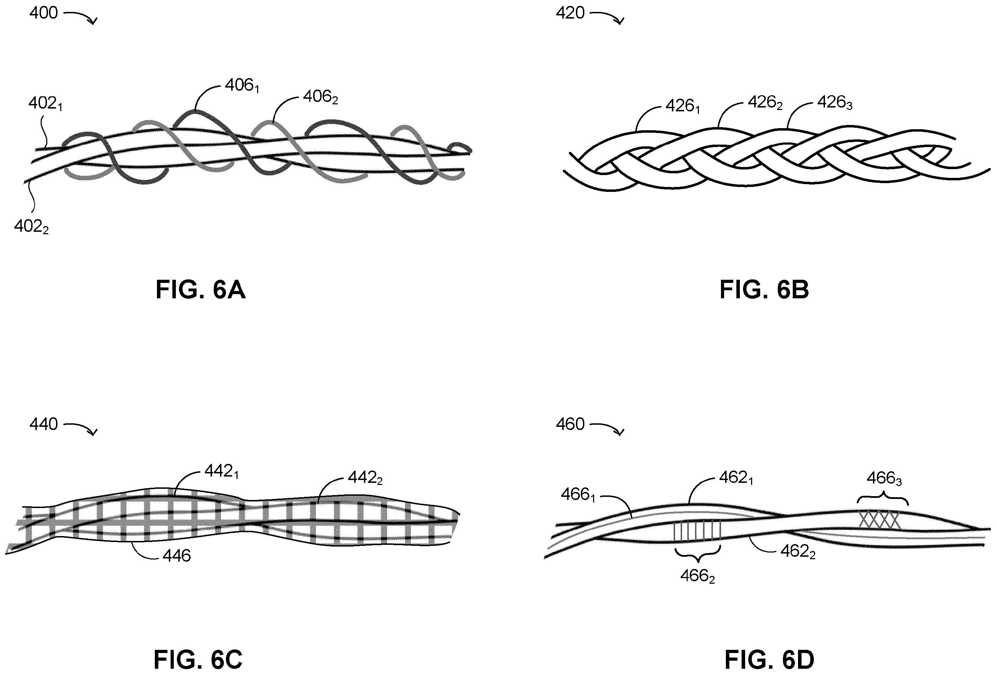

Alternative configurations (not shown) for drug dispensing wire 106 include adjoining to (e.g., wound helically around) only one of struts 102A and 102B. Another alternative configuration involves having a plurality of drug dispensing wires, where each wire is embedded with a different drug or the same drug. In the case of at least two different drug dispensing wires, each wire is embedded with a different drug having a different time-release profile. Alternatively, two drug dispensing wires can be embedded with the same drug but with different time-release profiles (linear, non-linear, burst, etc.). Also, the different drug dispensing wires may be composed of different materials. When at least two drug dispensing wires are intertwined with the struts, there may be multiple crossing points between the at least two drug dispensing wires. These crossing points can have knots or be unknotted. Another alternative is when an integer N > 2 of drug dispensing wires are intertwined or interlaced around the struts, they may form an N-braid pattern. This N-braid pattern can permute in the same manner (or differently) with respect to each one of the struts, as well as twist in the same orientation (or differently). An example three-strand braid configuration is described hereinbelow in conjunction with Figure 6B.

Once urethral implant 100 is delivered to a stricture of the subject's urethra (e.g., a constriction at the prostatic urethra), the implant expands to the expanded configuration, which is configured to apply continuous pressure to surrounding tissues of the urethra at the stricture, thereby expanding the stricture (e.g., reshaping and expanding the bladder neck and prostatic urethra). Anchor 104 expands to the expanded configuration and engages with surrounding tissues of the urethra, thereby anchoring urethral implant 100 in position. Anchor 104 facilitates urethral implant 100 to be relatively immobile (thus averting inadvertent migration into the bladder of the subject while being implanted). Urethral implant 100 is configured and operative to apply continuous pressure thereby expanding the internal cross-section at the stricture, thus increasing the ability of urine flow through the urethra. While urethral implant 100 is implanted, drug dispensing wire 106 is configured to release the drug at a controlled rate at the implantation site. Urethral implant 100 is enabled for removal from the urethra (via an implant extraction mechanism - not shown).

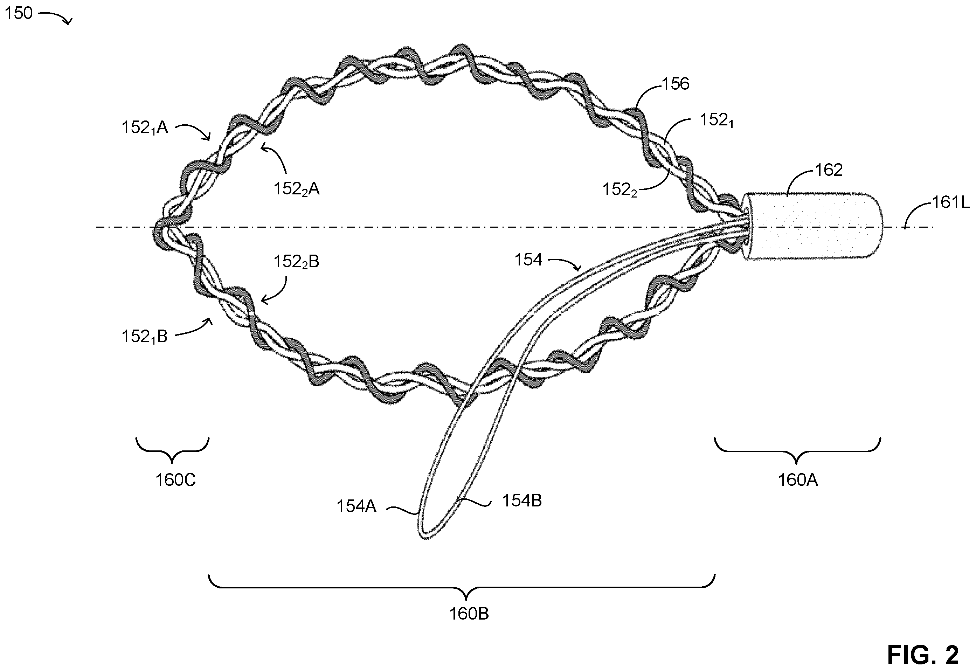

Reference is now made to Figure 2, which is a schematic illustration of the urethral implant, generally referenced 150, shown in an expanded configuration, having twisted wires, constructed and operative in accordance with another embodiment of the disclosed technique. Urethral implant 150 is generally similar to urethral implant 100 of Figures 1A-1C in structure and function, apart from urethral implant 150 including two twisted or intertwined wires (i.e., a twisted wire pair) instead of only one wire compared to urethral implant 100.

Specifically, urethral implant 150 includes two wires 152₁ and 152₂, an anchor 154, a drug dispensing wire 156, and a proximal cap 162. In an alternative configuration (not shown), proximal cap 162 is optional. Wires 152₁ and 152₂ are joined together (helically twisted or intertwined together), and each wire includes two strut sections extending longitudinally along a proximal portion 160A (interchangeably denoted herein "proximal end"), a central portion 160B (interchangeably denoted herein "mid-section"), and a distal portion 160C (interchangeably denoted herein "distal end"), and generally along a longitudinal axis 161L of urethral implant 150. Particularly, wire 152, includes two strut sections 152₁A and 152₁B, and wire 152₂ includes two strut sections 152₂A and 152₂B. Struts 152₁A and 152₁B of wire 152, are continuous (i.e., made of a single piece of wire) and converge at proximal end 160A. Similarly, struts 152₂A and 152₂B of wire 152₂ are continuous (i.e., made of a single piece of wire) and converge at proximal end 160A. In an alternative configuration (not shown), 152₁A and 152₁B of wire 152, are separate pieces that are mechanically joined together at distal end 160C and converge at proximal end 160A. In a similar alternative configuration (not shown), struts 152₂A and 152₂B of wire 152₂ are separate pieces that are mechanically joined together at distal end 160C and converge at proximal end 160A. Central portion 160B is defined to be between proximal end 160A and distal end 160C. Wires 152₁ and 152₂ are similar to wire 102 of urethral implant 102 in function. Likewise, anchor 154, and proximal cap 162 are similar in structure and function to anchor 104 and proximal cap 112 (respectively) of urethral implant 100 (Figs. 1A-1C).

Urethral implant 150 is compressible between an expanded configuration and a compressed configuration (similarly to urethral implant 100). Wires 152₁ and 152₂ generally have the same structural properties (e.g., equal length, thickness, cross-section), as well as the same material properties (e.g., made of the same material and having the same mechanical properties such as elasticity, strength, malleability, resilience, and the like). An alternative configuration (not shown) is where wires 152, and 152₂ are dissimilar with respect to at least one attribute such as structurally, geometrically (e.g., length, thickness, curvature), and materially different (i.e., not made of exactly the same material or have different mechanical properties such as flexibility, strength, and the like). Wires 152₁ and 152₂ are generally made of medical-grade bio-compatible materials (similar to wire 102).

Anchor 154 is identical to anchor 104 of Figs. 1A-1C. Anchor is a continuous wire structure that includes two sides embodied as two struts 154A and 154B, which extend outwardly from proximal end 160A into central portion 160B, to one side of urethral implant 150 of longitudinal axis 161L. Struts 154A and 154B are generally made from a single continuous piece of wire converging at proximal end 160A. Alternatively, struts 104A and 104B are separate pieces (not shown) that are mechanically joined together at an apex (i.e., a tip of anchor 104). Anchor 154 is flexible between the compressed configuration and the expanded configuration. Anchor 104 has the capability to self-expand into the expanded configuration once released from a delivery sheath (e.g., sheath 114 of Figure 1C). Anchor 154 is generally arcuate defining a closed shape (e.g., closed loop) having an internal hole and bilateral symmetry of the two struts. Anchor 154 is constructed and operative to engage with, and attach (anchor) to tissues of the urethra of the subject in the expanded configuration. Anchor 154 can be detached from tissues when urethral implant 150 is removed from the subject. Anchor 154 is made of the same material of wires 152₁ and 152₂. Alternatively, anchor 154 is not made of exactly the same materials of 152₁ and 152₂.

Drug dispensing wire 156 is similar to drug dispensing wire 106 of Figs. 1A-1C, in structure, composition, and function. Drug dispensing wire 156 is adjoined to (e.g., continuously and helically wound around) wires 152₁ and 152₂ along the length of the wire's corresponding struts 152₁A and 152₁B for wire 152₂A and 152₂B for wire 152₂. Drug dispensing wire 156 starts as well as ends at proximal end 160A (i.e., such that its two ends terminate at proximal end 160A). Drug dispensing wire 106 is adjoined to the struts of wires 152₁ and 152₂ by helically winding circumferentially and along their entire length. An alternative coupling configuration between drug dispensing wire 156 and the closed-shaped wires is a parallel coupling (i.e., the wires extending in parallel to each other, i.e., non-helically). Drug dispensing wire 156 has an overall length that is greater than the length of each wire 152, and 152₂, and according to one configuration it is less than the length of wires 152₁ and 152₂ combined. According to another configuration (not shown) drug dispensing wire 156 can be tightly helically coiled such that its overall length is greater than the length of wires 152₁ and 152₂ combined. Drug dispensing wire 156 is embedded with the same drug or drugs as drug dispensing wire 106. The drug functions to dispense (i.e., be released) into the urethra when urethral implant 150 is implanted within the subject. Once drug dispensing wire 156 is implanted, the drug is configured to gradually dissolve at a controlled rate according to its characteristic time-release drug profile. Drug dispensing wire 156 is made of a single strand or multiple strands of a material (e.g., fibrous) that can absorb as well as exude the embedded drug when urethral implant 150 is implanted. Drug dispensing wire 156 is compressible between the compressed configuration and expanded configuration of urethral implant 150.

When urethral implant 150 is implanted in the subject, it facilitates expansion of a stricture within the urethra of the subject, by applying pressure to surrounding tissues. Urethral implant 150 has two wires 152₁ and 152₂ (as opposed to one wire in urethral implant 100), enabling it to apply more force per area (pressure) to surrounding tissue than that of urethral implant 100. Urethral implant 150 is capable of storing more mechanical energy when compressed than urethral implant 100. Urethral implant 150 is compressible between two extremities: a fully compressed configuration and a fully expanded configuration, where continuous intermediate compression states are viable between these two extremities.

Urethral implant 150 provides a synergistic effect between the mechanical action of wires 152₁ and 152₂, and the therapeutic action of drug dispensing wire 156 that extends along the wire-to-tissue engaging area. Specifically, wires 152₁ and 152₂ mechanically engage and apply pressure to urethral tissues at the wire-to-tissue engaging area, while simultaneously drug dispensing wire 156 makes contact with the same urethral tissues as wires 152₁ and 152₂ and delivers the drug to those same tissues (i.e., targeted drug delivery).

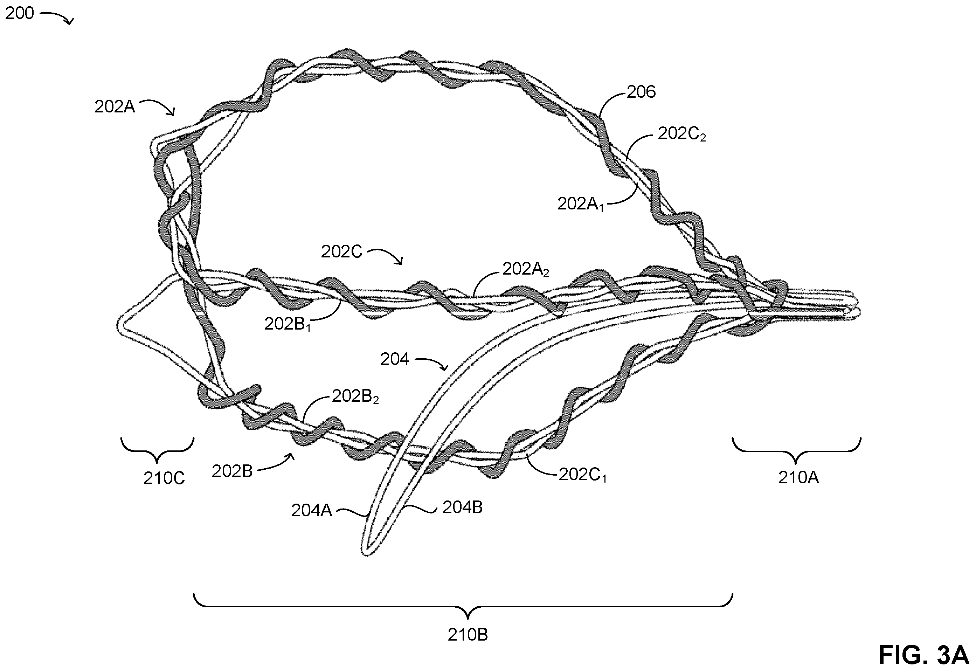

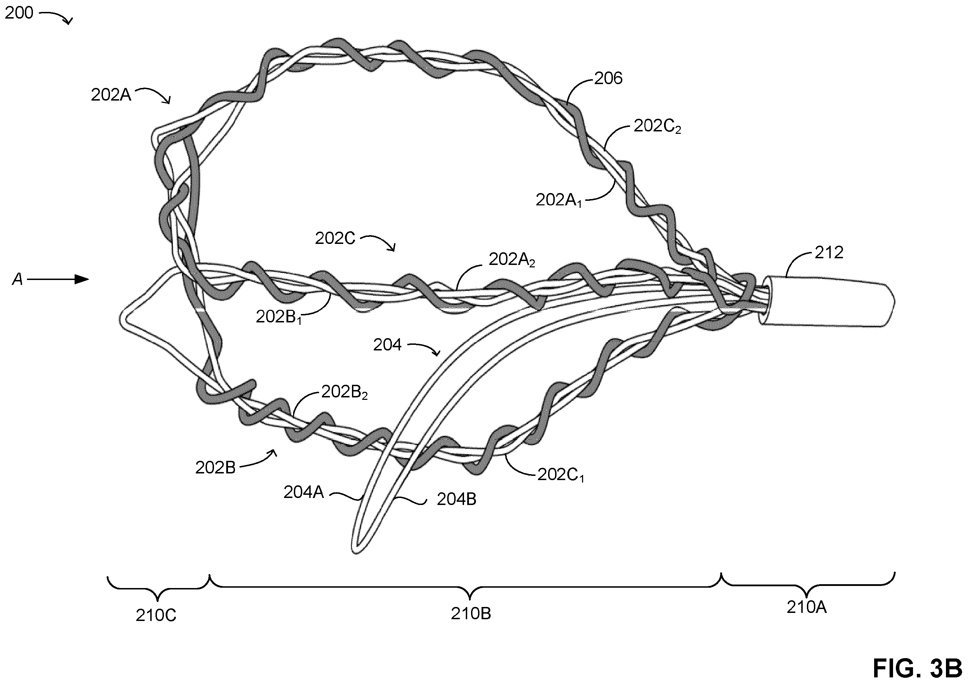

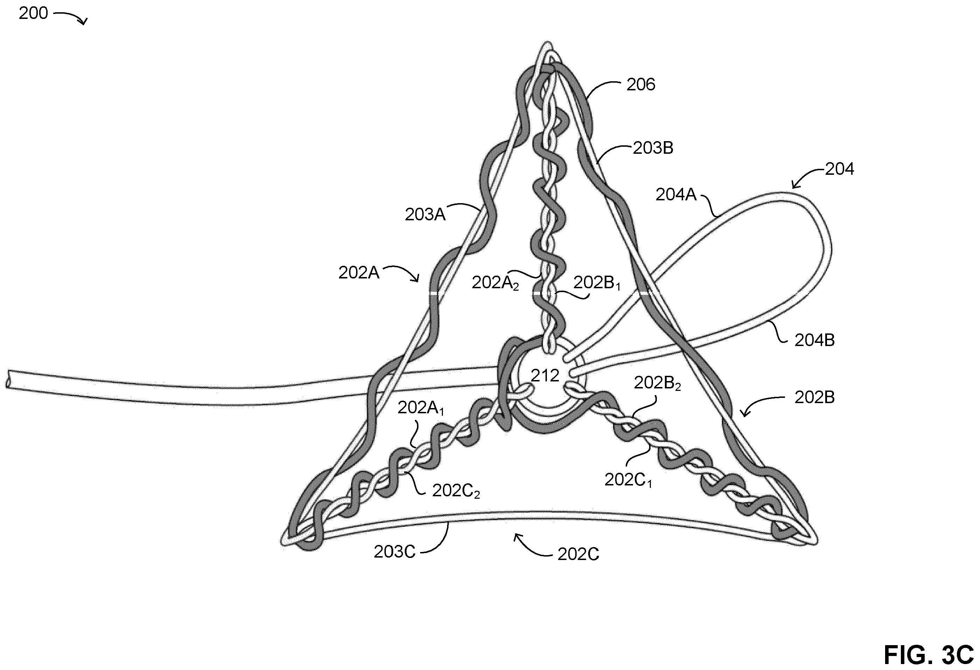

Reference is now made to Figures 3A, 3B, and 3C. Figure 3A is a schematic illustration of a urethral implant, generally referenced 200, shown in an expanded configuration, constructed and operative in accordance with a further embodiment of the disclosed technique. Figure 3B is a schematic illustration of the urethral implant of Figure 3A including a proximal cap, constructed and operative in accordance with the embodiment of the disclosed technique. Figure 3C is a schematic illustration of the urethral implant of Figure 2B shown from a distal-to-proximal direction A, constructed and operative in accordance with the embodiment of the disclosed technique.

Urethral implant 200 is generally constructed from a wireframe structure that includes three categories of closed-shape sub-structures having different form and function. A first category sub-structure is a mechanical structural framework of urethral implant 200 that includes three closed-shapes for engaging with urethral tissue, incising the urethral tissue, and expanding the urethral tissue at a particular urethral location where a stricture occurs (i.e., a constriction). A second category sub-structure is an anchor that includes at least one closed-shape structure for engaging with urethral tissue and anchoring urethral implant 200 in position. A third category sub-structure includes at least one drug dispensing wire that is embedded with a drug for dispensing within the urethra of the subject. Urethral implant 100 defines a proximal section 210A, a central section 210B, and a distal section 210C.

Specifically, urethral implant 200 includes three closed-shaped wires 202A, 202B, and 202C (i.e., the first category sub-structure, which is the wireframe structure), an anchor 204 (i.e., the second category sub-structure), and a drug dispensing wire 206 (i.e., a third category sub-structure), as shown in Figures 3A-3C. According to another configuration, urethral implant 200 includes a proximal cap 212 at proximal end 210A, as shown in Figures 3B and 3C. According to a minimal configuration shown in Figure 3A, proximal cap 212 is absent (i.e., optional).

Each closed-shaped wire includes two longitudinal sections extending between proximal end 210A and distal end 210C. Each closed-shape wire may include a lateral section between the two lateral sections. Specifically, closed-shape wire 202A includes longitudinal sections 202A, and 202A₂, and a lateral section 203A connecting between longitudinal sections 202A₁ and 202A₂ (as shown in Figure 3C). Closed-shape wire 202B includes longitudinal sections 202B, and 202B₂, and a lateral section 203B connecting between longitudinal sections 202B₁ and 202B₂ (as shown in Figure 3C). Closed-shape wire 202C includes longitudinal sections 202C₁ and 202C₂, and a lateral section 203C connecting between longitudinal sections 202C, and 202C₂ (as shown in Figure 3C). Each longitudinal section of a closed-shaped wire is adjoined along its length with another longitudinal section of another one closed-shaped wire. Specifically, longitudinal section 202A₂ of closed-shaped wire 202A is adjoined along its length with longitudinal section 202B₁ of closed-shaped wire 202B, longitudinal section 202B₂ of closed-shaped wire 202B is adjoined along its length with longitudinal section 202C, of closed-shaped wire 202C, and longitudinal section 202A₂ of closed-shaped wire 202A is adjoined along its length with longitudinal section 202C₂ of closed-shaped wire 202C. According to the preferred configuration, each closed-shape is constructed from a single continuous wire that defines at least two longitudinal sections. Alternatively, there are three sections (a lateral section between two longitudinal sections). As shown in Figures 3A-3C, the method of adjoining the wires is by helically twisting one around the other (longitudinal sections), as a twisted or intertwined wire pair where one wire is twists clockwise while the other wire twists counterclockwise (left/right-handed twists). In a preferred configuration, the wires are composed of the same material, and have the same physical attributes (e.g., width, cross-section). In an alternative configuration (not shown), the wires are not the same in at least one respect (e.g., composition, physical attribute, etc.).

With particular reference to Figure 3C, urethral implant 200 forms a triangular pyramidal structure having a triangular base that is formed from lateral sections 203A, 203B, and 203C at distal end 210C, and an apex at proximal end 210A where longitudinal wires converge (at proximal cap 212 shown in Figures 3B and 3C and without a proximal cap shown in Figure 3A). Proximal cap 212 covers the ends (termini) of the first, second, and third category wires at proximal section 210A as shown in Figures 3B and 3C. Specifically, proximal cap 112 covers the ends (termini) of wires 202A, 202B, 202C, the ends of anchor 204, as well the ends of drug dispensing wire 206, at proximal section 120A (Figs. 3B and 3C). In an alternative configuration (not shown), wires 202A, 202B, and 202C are closed curves (i.e., having no endpoints).

Anchor 204 is identical to anchor 104 of Figures 1A-1C, and anchor 154 of Figure 2. Anchor 204 is a continuous wire structure that includes two sides embodied as two struts 204A and 204B, which extend outwardly from proximal end 210A into central portion 210B, to one side of urethral implant 200 and terminate at a terminus (i.e., the tip, apex of anchor 204). Anchor 204 generally originates from proximal end 210A, passes through one of the closed-shaped wires (e.g., closed-shaped wire 202B as shown in Figures 3B and 3C). Struts 204A and 204B are generally made from a single continuous piece of wire that converges at two points, namely the origin, i.e., proximal end 210A and the terminus of anchor 204. In an alternative configuration struts 204A and 204B are separate pieces (not shown) that are mechanically joined together at the apex or tip of anchor. In another alternative configuration (not shown), anchor 204 is a closed curve (i.e., having no endpoints). Anchor 204 is flexible between the compressed configuration and the expanded configuration. Anchor 204 has the capability to self-expand into the expanded configuration once released from a delivery sheath (e.g., sheath 114 of Figure 1C). Anchor 204 is generally arcuate defining a closed shape (e.g., closed loop) having an internal hole and bilateral symmetry of the two struts. When in the expanded configuration, anchor 204 extends beyond the width of the closed-shaped wires, thereby effectively widening the total width of urethral implant 200. Anchor 204 is constructed and operative to engage with, and attach (anchor) to tissues of the urethra of the subject in the expanded configuration. Anchor 204 can be detached from tissues when urethral implant 204 is removed from the subject. Anchor 204 is made of the same material as that of the closed-shaped wires. Alternatively, anchor 204 is not made of exactly the same materials that compose the closed-shaped wires.

Drug dispensing wire 206 is similar to drug dispensing wire 106 of Figs. 1A-1C, and drug dispensing wire 156 of Fig. 2 in structure, composition, and function. Drug dispensing wire 206 is coupled to (i.e., adjoined continuously and helically wound around) wires longitudinal sections 202A₁ and 202A₂ of closed-shaped wire 202A, longitudinal sections 202B₁ and 202B₂ of closed-shaped wire 202B, and longitudinal sections 202C₁ and 202C₂ of closed-shaped wire 202C. Furthermore, drug dispensing wire 206 is adjoined to (e.g., helically wound around) lateral sections 203A and 203B but not adjoined along the length of lateral section 203C, as distinctly shown in Figure 3C. In an alternative configuration (not shown), drug dispensing wire 206 is adjoined to lateral section 203C (i.e., adjoined to all longitudinal and lateral wires). Drug dispensing wire 206 is continuous and originates as well as terminates at proximal end 210A (i.e., such that its two ends terminate at proximal end 210A). Drug dispensing wire 206 is helically wounded circumferentially and along the entire length of the longitudinal sections of each of the closed-shaped wires. The twisting or intertwining method of drug dispensing wire 206 around the longitudinal sections can be clockwise or counterclockwise (e.g., left or right handed twisting). The overall length of drug dispensing wire 206 is greater than the length of each closed-shaped wire. Drug dispensing wire 206 is compressible between the compressed configuration and the expanded configuration of urethral implant 200.

Drug dispensing wire 206 is embedded with the same drug or drugs as embedded in drug dispensing wire 106. The drug is dispensed (i.e., be released, administered) into the urethra when urethral implant 200 is implanted within the subject. Once drug dispensing wire 206 is implanted along with urethral implant 200, the drug gradually dissolves at a controlled rate according to its characteristic time-release drug profile. The drug includes a chemical substance that is configured to produce or perform a biological effect on the subject. Drug dispensing wire 206 is made of a single strand or multiple strands of a material (e.g., fibrous) that can absorb as well as exude the embedded drug when urethral implant 200 is implanted.

Alternative configurations (not shown) for drug dispensing wire 206 include adjoining to (e.g., wound helically around) only one of the longitudinal sections. Another alternative configuration involves having a plurality of drug dispensing wires, where each wire is embedded with a different drug or the same drug. In the case of at least two different drug dispensing wires, each wire is embedded with a different drug having a different time-release profile. Alternatively, two drug dispensing wires can be embedded with the same drug but with different time-release profiles (linear, non-linear, burst, etc.). Also, the different drug dispensing wires may be composed of different materials.

Urethral implant 200 (including closed-shape wires 202A, 202B, and 202C, anchor 204, and drug dispensing wire 206) is compressible between an expanded configuration and a compressed configuration (similarly to urethral implants 100 and 150). Closed-shape wires 202A, 202B, and 202C generally have the same structural properties (e.g., dimensions such as length, thickness, cross-section), as well as the same material properties (e.g., made of the same material and having the same mechanical properties such as elasticity, strength, malleability, resilience, and the like). An alternative configuration (not shown) is where wires 202A, 202B, and 202C are dissimilar with respect to at least one attribute such as structurally, geometrically (e.g., length, thickness, curvature), and materially different (i.e., not made of exactly the same material or have different mechanical properties such as flexibility, strength, and the like). Wires 202A, 202B, and 202C are generally made of medical-grade bio-compatible materials (similar to wire 102 and wires 152₁ and 152₂ of the preceding embodiments).

Following implantation of urethral implant 200 in the subject, the implant facilitates expansion of a stricture within the urethra, by applying pressure to surrounding tissues. The dual intertwined wires (a twisted wire pair) of urethral implant 200 are capable of storing more mechanical energy when in a compressed state than that of single-wire urethral implant 100. Urethral implant 200 is generally compressible between two extremities: a fully compressed configuration and a fully expanded configuration, where continuous intermediate compression states are viable between the two extremities. According to an alternative configuration (not shown) the urethral implant can include more than two intertwined wires (e.g., longitudinally) such as a wire bundle.

Urethral implant 200 provides a synergistic effect between the mechanical action of the closed-shaped wires 202A, 202B, and 202C and the therapeutic action of drug dispensing wire 206 that extends along the wire-to-tissue engaging area. Specifically, the longitudinal sections 202A₁, 202A₂, 202B₁, 202B₂, 202C₁, and 202C₂ of the closed-shaped wires mechanically engage and apply pressure to urethral tissues at the wire-to-tissue engaging area, while simultaneously drug dispensing wire 206 makes contact with the same urethral tissues as the longitudinal sections of the closed-shaped wires and delivers the drug to those same tissues (i.e., targeted drug delivery).

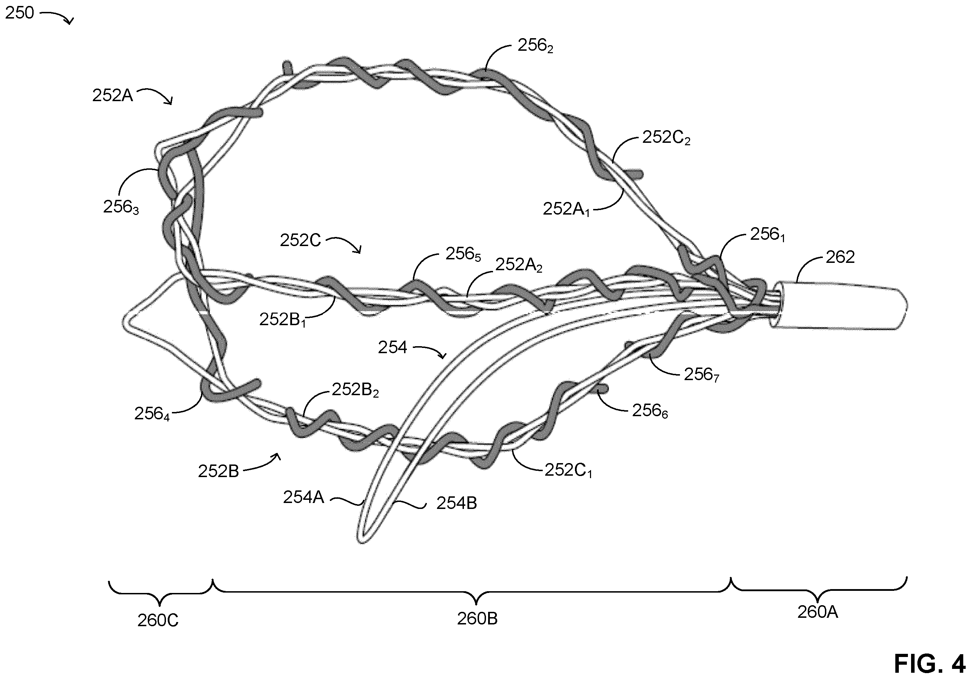

Reference is now made to Figure 4, which is a schematic illustration of a urethral implant, generally referenced 250, having a plurality of discontinuous drug dispensing wires, constructed and operative in accordance with another embodiment of the disclosed technique. Urethral implant 250 is the same as urethral implant 200 of Figures 3A-3C except for the drug dispensing wire having a plurality of discontinuous sections. Urethral implant 250 defines a proximal section 260A, a central section 260B, and a distal section 260C. Specifically, urethral implant 250 includes three closed-shaped wires 252A, 252B, and 252C, an anchor 254, a proximal cap 262, and a plurality of drug dispensing wires 256₁, 256₂, 256₃, 256₄, 256₅, 256₆, and 256₇. In general, urethral implant 250 can include a plurality of *N* drug dispensing wires 256₁,...,256*_{N}* (where *N* is a positive integer greater than 1). In particular, and without loss of generality, urethral implant 250 includes seven separate and discontinuous drug dispensing wires (as an example), each of different lengths, and each of which is coupled to at least part of one closed-shaped wires 252A, 252B, and 252C. Specifically, drug dispensing wire 256, is coupled to wires 252A₁ and 252C₂, drug dispensing wire 256₂ is coupled to wires 252A, and 252C₂, drug dispensing wire 256₃ is coupled to wires 252A₁, 252C₂, 252B₁ and 252A₂, drug dispensing wire 256₄ is coupled to wires 252A₁, 252C₂, 252B₂ and 252C₁, drug dispensing wire 256₅ is coupled to wires 252A₂, and 252B₁, drug dispensing wire 256₆ is coupled to wires 252B₂ and 252C₁, and drug dispensing wire 256₇ is coupled to wires 252B₂, 252C₁, 252A₁, and 252C₂. In an alternative configuration (not shown) to the configuration shown in Figure 4, drug dispensing wires 256₁,...,256₇ are of equal lengths. In one configuration, drug dispensing wires 256₁,...,256₇ are the same and have the same characteristics, namely, are embedded with the same drug composition, and the same drug time-release profile, physical attributes (e.g., length, width, cross-section, curvature, torsion, etc.). In another configuration, at least one of drug dispensing wire of wires 256₁,...,256₇ is different with respect to the other drug dispensing wires in at least one characteristic, such as drug composition, drug time-release profile, mechanism of action (MOA), physical attributes, and the like. For example, drug dispensing wire 256₁ is embedded with the drug sirolimus, and drug dispensing wire 256₃ is embedded with the drug everolimus. Generally, different combinatorial combinations of different characteristics of different drug dispensing wires are possible.

Closed-shaped wires 252A, 252B, and 252C, as well as anchor 254, and proximal cap 262 are the same in structure and function as closed-shaped wires 202A, 202B, and 202C, anchor 204, and proximal cap 212 (respectively), described in conjunction with Figures 3A-3C. The description of elements with respect to Figures 3A-3C applies to those same elements of urethral implant 250 of Figure 4.

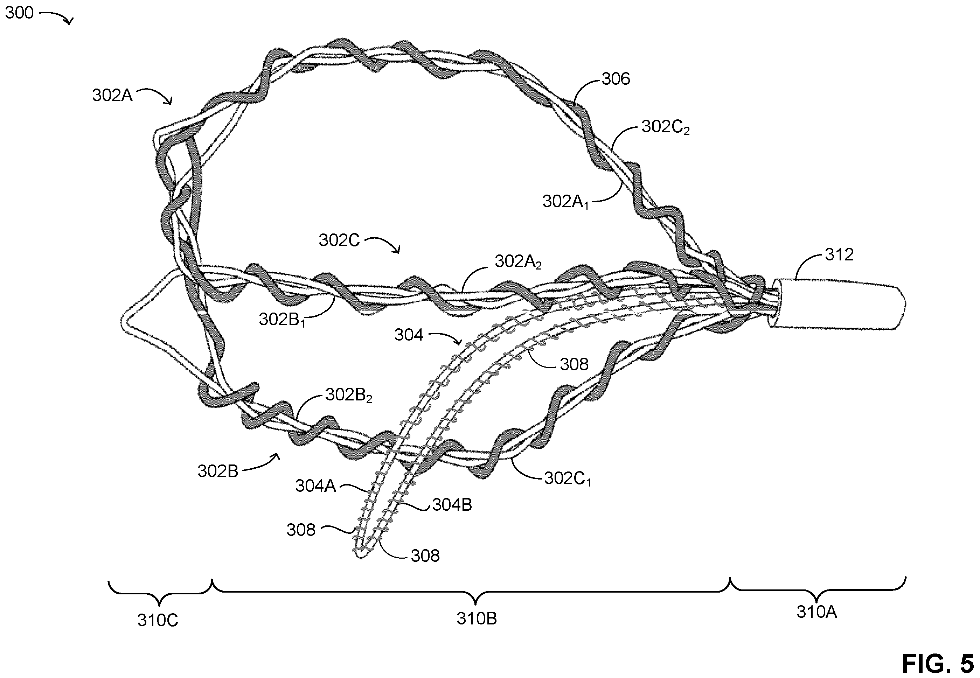

Reference is now made to Figure 5, which is a schematic illustration of a urethral implant, generally referenced 300, having drug dispensing wires coupled to closed-shaped wires that include an anchor, constructed and operative in accordance with a further embodiment of the disclosed technique. Urethral implant 300 is the same as urethral implant 200 of Figures 3A-3C except for an additional drug dispensing wire coupled to the anchor. Specifically, urethral implant 300 includes three closed-shaped wires 302A, 302B, and 302C, an anchor 304, a drug dispensing wire 306, a drug dispensing wire 308, and a proximal cap 312. Urethral implant 300 defines a proximal section 310A, a central section 310B, and a distal section 310C. Anchor 304 is identical to anchor 204 of Figures 3A-3C. Anchor 304 is a continuous wire structure that includes two sides embodied as two struts 304A and 304B, which extend outwardly from proximal end 310A into central portion 310B, to one side of urethral implant 300 and terminate at a terminus (i.e., the tip, apex of anchor 304). Struts 304A and 304B are generally made from a single continuous piece of wire that converges at two points, namely the origin, i.e., proximal end 310A and the terminus of anchor 304. Anchor 304 is flexible between the compressed configuration and the expanded configuration. Anchor 304 has the capability to self-expand into the expanded configuration. Anchor 304 is generally arcuate defining a closed shape (e.g., closed loop) having an internal hole and bilateral symmetry of the two struts 304A and 304B.

Drug dispensing wire 308 is coupled to (i.e., adjoined continuously and helically wound around) struts 304A and 304B of anchor 304. Drug dispensing wire 308 and anchor 304 are compressible between the compressed configuration and the expanded configuration of urethral implant 300. In one configuration, drug dispensing wire 308 is different than drug dispensing wire 306. Differences include different structure, composition, type of drug embedded, drug time-release profile, function, and the like. For example, drug dispensing wire 308 is composed of a fiber or fiber strand that is thinner and shorter than that of drug dispensing wire 306, as illustrated in Figure 5. Furthermore, drug dispensing wire 308 can be embedded with a different drug than that of drug dispensing wire 306 (e.g., non-steroidal anti-inflammatory drugs (NSAIDS), etc.). In another alternative configuration (not shown), drug dispensing wire 308 is the same as drug dispensing wire 306 in terms of structure, composition, function, and the like. In another alternative configuration (not shown), each one of struts 304A and 304B has coupled therewith a different drug dispensing wire. In such a configuration, their differences can be manifested by at least one differentiating characteristic (e.g., with respect to structure, composition, drug embedding, mechanism of action (MOA), etc.).

Closed-shaped wires 302A, 302B, 302C, as well as anchor 304, drug dispensing wire 306, and proximal cap 312 are the same in structure and function as closed-shaped wires 202A, 202B, and 202C, anchor 204, and proximal cap 212 (respectively), as described in conjunction with Figures 3A-3C. The description of elements with respect to Figures 3A-3C applies to those same elements of urethral implant 300 of Figure 5.

Reference is now made to Figures 6A, 6B, 6C, and 6D, which are schematic illustrations of various configurations of drug dispensing wires, constructed and operative in accordance with the embodiments of the disclosed technique. The wires of the urethral implant wireframe structure as well as the drug dispensing wires described hereinbelow in conjunction with Figures 6A-6D are respectfully the same as the wires of the urethral implant wireframe structure and drug dispensing wires heretofore described in the previous embodiments in conjunction with Figures 3A-3C, 4, and 5.

Figure 6A is schematic illustration showing a double helix configuration, generally referenced 400, of two drug dispensing wires helically wound about two wires of the urethral implant of the disclosed technique. Specifically, Figure 6A illustrates two urethral implant wires 402, and 402₂ as well as two drug dispensing wires 406, and 406₂ (only partially shown, i.e., not their full lengths). Particularly, drug dispensing wires 406, and 406₂ are embodied as two helices in a double helix configuration, helically wound about wires 402, and 402₂ (i.e., coupled therewith). Drug dispensing wires 406, and 406₂ are identical or congruent (differing only by a translation or position along wires 402, and 402₂) (i.e., they have the same geometry, same composition, embedded with the same drug, etc.). Alternatively, drug dispensing wires 406, and 406₂ are different in at least one respect (e.g., structurally different, embedded with different drugs, composed of different materials, have different time-release profiles, etc.).

Figure 6B is a schematic illustration showing a three-strand braid configuration, generally referenced 420, including three drug dispensing wires for winding about two wires of the urethral implants of the disclosed technique. Specifically, Figure 6B illustrates three drug dispensing wires 426₁, 426₂, and 426₃ (only partially shown, i.e., not their full lengths) for winding about the wireframe structure (not shown) of the urethral implants of the disclosed technique. In one configuration, drug dispensing wires 426₁, 426₂, and 426₃ are identical (differing only by a translation or position along wires) (i.e., they have the same geometry, same composition, embedded with the same drug, etc.). Alternatively, at least one drug dispensing wire 426₁, 426₂, and 426₃ is different in at least one respect to at least one other drug dispensing wire (e.g., structurally different, embedded with different drugs, composed of different materials, have different time-release profiles, etc.).

Figure 6C is a schematic illustration showing a drug dispensing fabric configuration, generally referenced 440, covering two wires of the urethral implant of the disclosed technique. Specifically, Figure 6C illustrates wires 442₁, and 442₂ of the urethral implant, and a drug dispensing fabric 446 that covers the wires (only partially shown, i.e., not their full lengths). Drug dispensing fabric 446 is embedded with a drug according to the principles heretofore described (e.g., drug dispensing wire 306). Fabric 446 can be woven or nonwoven (e.g., bonded together). According to one configuration, drug dispensing fabric 446 is a cloth (e.g., cotton, polyester) that is embedded with a drug that can be dispensed when implanted within the body of the subject. According to an alternative configuration, fabric 446 is a material other than cloth (e.g., polymer) that is woven into a pattern. Multiple materials can be interwoven into various patterns (e.g., plain weave, etc.).

Figure 6D is a schematic illustration, showing two different drug dispensing wire configurations, generally referenced 460, for two wires of the urethral implants of the disclosed technique. Specifically, Figure 6D illustrates wires 462₁, and 462₂ of the urethral implant, and three drug dispensing wire groups. The first drug dispensing wire group includes one drug dispensing wire 466, coupled to and extending in parallel to wire 462₁. A second drug dispensing wire group includes a plurality of discrete drug dispensing wires 466₂ coupled to and extending perpendicularly to the direction of extension of wire 462₂, partially along the length of wire 462₂. A third drug dispensing wire group includes a plurality of discrete drug dispensing wires 466₃ coupled to and extending in an X-pattern or crisscross pattern along the direction of extension of wire 462₂, partially along the length of wire 462₂. Alternative configurations (not shown) are viable (e.g., oblique pattern, O-pattern, V-pattern, etc.). It will be appreciated by persons skilled in the art that the disclosed technique is not limited to what has been particularly shown and described hereinabove.

## Claims

1. An implant (100; 150; 200; 250; 300; 400) for the urethra of a subject, the implant (100; 150; 200; 250; 300; 400) comprising:
at least two struts (102A; 102B; 152₁A, 152₁B, 152₂A, 152,B; 202A, 202B, 202C; 252A, 252B, 252C; 302A, 302B, §02C; 402₁, 402₂; 462₁, 462₂) extending longitudinally between a proximal end and a distal end of said implant, each of said struts (102A; 102B; 152₁A, 152₁B, 152₂A, 152,B; 202A, 202B, 202C; 252A, 252B, 202C; 302A, 302B, 302C; 402₁, 402₂; 462₁, 462₂) being flexible between a compressed configuration that enables delivery of said implant (100; 150; 200; 250; 300; 400) to a stricture in said urethra, and an expanded configuration that facilitates expansion of said stricture; an anchor (104; 154; 204; 254; 304) extending outwardly from at least one of said proximal end an said distal end, for anchoring said implant (100; 150; 200; 250; 300; 400), **characterized by** at least one drug dispensing wire (106; 156; 206; 256; 306; 406; 426; 466; fabric 446), adjoined to and extending longitudinally along, at least one of said struts (102A; 102B; 152₁A, 152₁B, 152₂A, 152₁B; 202A, 202B, 202C; 252A, 252B, 252C; 302A, 302B, 302C; 402₁, 402₂; 462₁, 462₂), said drug dispensing wire (106; 156; 206; 256; 306; 406; 426; 466; fabric 446) is embedded with a drug for releasing into said urethra.

2. The implant according to claim 1, wherein each one of: said at least two struts (102A; 102B; 152₁A, 152₁B, 152₂A, 152,B; 202A, 202B, 202C; 252A, 252B, 252C; 302A, 302B, 302C; 402₁, 402₂; 462₁, 462₂), said anchor (104; 154; 204; 254; 304), and
said at least one drug dispensing wire (106; 156; 206; 256; 306; 406; 426; 466; fabric 446), is respectively a continuous wire, and / or
further comprising a proximal cap (112; 162) located at said proximal end, said proximal cap (112; 162; 212; 262; 312) is configured to cover ends of each said continuous wire of said at least two struts (102A; 102B; 152₁A, 152₁B, 152₂A, 152,B; 202A, 202B, 202C; 252A, 252B, 252C; 302A, 302B, 302C; 402₁, 402₂; 462₁, 462₂), said anchor (104; 154; 204; 254; 304), and said at least one drug dispensing wire (106; 156; 206; 256; 306; 406; 426; 466; fabric 446).

3. The implant according to claim 1, wherein said implant (100; 150; 200; 250; 300; 400) is configured for applying pressure on tissues of said prostatic urethra, and / or wherein said at least one drug dispensing wire (106; 156; 206; 256; 306; 406; 426; 466; fabric 446) is embedded with said drug selected from a list consisting of: sirolimus (rapamycin); and everolimus, and / or
wherein said at least one drug dispensing wire (106; 156; 206; 256; 306; 406; 426;) is helically wound around at least one of said struts, and / or
wherein said at least one drug dispensing wire (106; 156; 206; 256; 306; 406; 426; 466; fabric 446) includes a plurality of drug dispensing wires (106; 156; 206; 256; 306; 406; 426; 466₁, 466₂, 466₃), where each drug dispensing wire is embedded with a different drug, and / or
wherein said at least one drug dispensing wire is made of at least on single strand of material (fabric 446) that is configured to absorb as well as exude said drug, and / or
wherein said at least one drug dispensing wire (106; 156; 206; 256; 306; 406; 426; 466; fabric 446) is configured to dissolve at a controlled rate according to its characteristic time-release profile, after said implant is implanted in said subject.

4. The implant according to claim 1, wherein each of said at least two struts includes a twisted wire pair (152₁A, 152,B, 152₂A, 152,B; 202A, 202B, 202C; 252A, 252B, 252C; 302A, 302B, 302C; 402₁, 402₂; 462₁, 462₂) extending longitudinally between said proximal end and said distal end.

5. The implant according to claim 1, wherein said implant (100; 150; 200; 250; 300; 400) is configured to be enfolded in a sheath (114) in said compressed configuration, and / or
wherein said implant (100; 150; 200; 250; 300; 400) has intermediate compression states between said expanded configuration and said compressed configuration.

6. The implant according to claim 1, wherein said implant (100; 150; 200; 250; 300; 400) is configured for implantation in the prostatic urethra of said subject, and for applying pressure on surrounding tissues of said prostatic urethra in said open configuration.

7. The implant according to claim 1, wherein said anchor (104; 154; 204; 254; 304) is configured to prevent said implant (100; 150; 200; 250; 300; 400) from migrating toward a bladder of said subject.

8. The implant according to claim 1, wherein said implant (100; 150; 200; 250; 300; 400) is removable from the subject.

9. An implant for the urethra of a subject, the implant (200; 250; 300; 400) comprising:
three closed-shaped wires (202A, 202B, 202C; 252A, 252B, 252C; 302A, 302B, 302C), each closed-shape wire (202A, 202B, 202C; 252A, 252B, 252C; 302A, 302B, 302C) includes two longitudinal sections extending between a proximal end and a distal end of said implant, each longitudinal section of a closed-shaped wire (202A, 202B, 202C; 252A, 252B, 252C; 302A, 302B, 302C) is adjoined along its length with another said longitudinal section of another one of said closed-shaped wire (202A, 202B, 202C; 252A, 252B, 252C; 302A, 302B, 302C), said closed-shaped wires (202A, 202B, 202C; 252A, 252B, 252C; 302A, 302B, 302C) are configured to engage with and to expand urethral tissue at a stricture in said urethra; an anchor (204; 254; 304), including a closed-shape wire (204A, 204B; 254A, 254B; 304A, 304B), for anchoring said implant to said urethra, **characterized by** at least one drug dispensing wire (206; 256; 306), adjoined to and extending longitudinally along at least one of said closed-shaped wires (202A, 202B, 202C; 252A, 252B, 252C; 302A, 302B, 302C), said drug dispensing wire (206; 256; 306) is embedded with a drug for releasing to said urethra.

10. The implant according to claim 9, wherein each one of: said three closed-shaped wires (202A, 202B, 202C; 252A, 252B, 252C; 302A, 302B, 302C), said anchor (204; 254; 304), and said at least one drug dispensing wire (206; 256; 306), is respectively a continuous wire, and / or
further comprising a proximal cap (112; 162; 212; 262; 312) located at said proximal end, said proximal cap (112; 162; 212; 262; 312) is configured to cover ends of each said three closed-shaped wires (202A, 202B, 202C; 252A, 252B, 252C; 302A, 302B, 302C),, said anchor (204; 254; 304), and said at least one drug dispensing wire (206; 256; 306).

11. The implant according to claim 9, wherein said implant (200; 250; 300; 400) is configured for applying pressure on tissues of said prostatic urethra, and/or wherein said at least one drug dispensing wire (206; 256; 306) is embedded with said drug selected from a list consisting of: sirolimus (rapamycin); and everolimus, and / or wherein said at least one drug dispensing wire (206; 256; 306) is helically wound around at least one of said three closed-shaped wires (202A, 202B, 202C; 252A, 252B, 252C; 302A, 302B, 302C), and / or wherein said at least one drug dispensing wire (206; 256; 306) includes a plurality of drug dispensing wires (466₁, 466₂, 466₃), where each drug dispensing wire is embedded with a different drug, and / or
wherein said at least one drug dispensing wire is made of at least a single strand of material (fabric 446) that is configured to absorb as well as exude said drug and / or wherein said at least one drug dispensing wire (206; 256; 306; 406; 426; 466; fabric 446) is configured to dissolve at a controlled rate according to its characteristic time-release profile, after said implant is implanted in said subject, and / or
wherein said at least one drug dispensing wire includes at least two drug dispensing wires (206; 256; 306; 308), whereby at least one drug dispensing wire (206; 256; 306) is adjoined to at least one of said three closed-shaped wires (202A, 202B, 202C; 252A, 252B, 252C; 302A, 302B, 302C), and at least another drug dispensing wire (308) is adjoined to said anchor (204; 254; 304).

12. The implant according to claim 9, wherein said implant (200; 250; 300; 400) is configured to be enfolded in a sheath (114) in said compressed configuration, and / or wherein said implant (200; 250; 300; 400) has intermediate compression states between said expanded configuration and said compressed configuration.

13. The implant according to claim 9, wherein said implant (200; 250; 300; 400) is configured for implantation in the prostatic urethra of said subject, and for applying pressure an surrounding tissues of said prostatic urethra in said open configuration.

14. The implant according to claim 9, wherein said anchor (204; 254; 304) is configured to prevent said implant (200; 250; 300; 400) from migrating toward a bladder of said subject.

15. The implant according to claim 9, wherein said implant (200; 250; 300; 400) is removable from the subject.

## Patentansprüche

1. Implantat (100; 150; 200; 250; 300; 400) für die Harnröhre eines Subjekts, wobei das Implantat (100; 150; 200; 250; 300; 400) umfasst:
mindestens zwei Streben (102A; 102B; 152₁A, 152₁B, 152₂A, 152₁B; 202A, 202B, 202C; 252A, 252B, 252C; 302A, 302B, §02C; 402₁, 402₂; 462₁, 462₂), die sich in Längsrichtung zwischen einem proximalen Ende und einem distalen Ende des Implantats erstrecken, wobei die Streben (102A; 102B; 152₁A, 152₁B, 152₂A, 152₁B; 202A, 202B, 202C; 252A, 252B, 202C; 302A, 302B, 302C; 402₁, 402₂; 462₁, 462₂) zwischen einer komprimierten Auslegung, die die Abgabe des Implantats (100; 150; 200; 250; 300; 400) an eine Struktur in der Harnröhre ermöglicht, und einer expandierten Auslegung, die die Expansion der Struktur erleichtert, biegsam sind; einen Anker (104; 154; 204; 254; 304), der sich von mindestens einem von dem proximalen Ende und dem distalen Ende nach außen erstreckt, zum Verankern des Implantats (100; 150; 200; 250; 300; 400), **gekennzeichnet durch** mindestens einen arzneimittelabgebenden Draht (106; 156; 206; 256; 306; 406; 426; 466; Stoff 446), der an mindestens eine der Streben (102A; 102B; 152₁A, 152₁B, 152₂A, 152₁B; 202A, 202B, 202C; 252A, 252B, 252C; 302A, 302B, 302C; 402₁, 402₂; 462₁, 462₂) grenzt und sich in Längsrichtung entlang derselben erstreckt, wobei in den arzneimittelabgebenden Draht (106; 156; 206; 256; 306; 406; 426; 466; Stoff 446) ein Arzneimittel zur Freisetzung in die Harnröhre eingebettet ist.

2. Implantat nach Anspruch 1, wobei: die mindestens zwei Streben (102A; 102B; 152₁A, 152₁B, 152₂A, 152₁B; 202A, 202B, 202C; 252A, 252B, 252C; 302A, 302B, 302C; 402₁, 402₂ ; 462₁, 462₂), der Anker (104; 154; 204; 254; 304) und der mindestens eine arzneimittelabgebende Draht (106; 156; 206; 256; 306; 406; 426; 466; Stoff 446) jeweils ein kontinuierlicher Draht sind, und / oder
ferner eine proximale Kappe (112; 162) umfassen, die sich an dem proximalen Ende befindet, wobei die proximale Kappe (112; 162; 212; 262; 312) dazu ausgelegt ist, die Enden eines jeden kontinuierlichen Drahts von den mindestens zwei Streben (102A; 102B; 152₁A, 152₁B, 152₂A, 152₁B; 202A, 202B, 202C; 252A, 252B, 252C; 302A, 302B, 302C; 402₁, 402₂; 462₁, 462₂), dem Anker (104; 154; 204; 254; 304) und dem mindestens einen arzneimittelabgebenden Draht (106; 156; 206; 256; 306; 406; 426; 466; Stoff 446) abzudecken.

3. Implantat nach Anspruch 1, wobei das Implantat (100; 150; 200; 250; 300; 400) zum Aufbringen von Druck auf die Gewebe der Harnröhrenprothese ausgelegt ist, und / oder wobei in den mindestens einen arzneimittelabgebenden Draht (106; 156; 206; 256; 306; 406; 426; 466; Stoff 446) das Arzneimittel eingebettet ist, das aus einer Liste ausgewählt wurde, bestehend aus: Sirolimus (Rapamycin) und Everolimus, und / oder
wobei der mindestens eine arzneimittelabgebende Draht (106; 156; 206; 256; 306; 406; 426;) spiralförmig um mindestens eine der Streben gewunden ist, und / oder wobei der mindestens eine arzneimittelabgebende Draht (106; 156; 206; 256; 306; 406; 426; 466; Stoff 446) eine Vielzahl von arzneimittelabgebenden Drähten (106; 156; 206; 256; 306; 406; 426; 466₁, 466₂, 466₃) aufweist, wobei in jeden arzneimittelabgebenden Draht ein anderes Arzneimittel eingebettet ist, und / oder
wobei der mindestens eine arzneimittelabgebende Draht aus mindestens einem einzelnen Strang von Material (Stoff 446) besteht, das dazu ausgelegt ist, das Arzneimittel zu absorbieren und abzusondern, und / oder
wobei der mindestens eine arzneimittelabgebende Draht (106; 156; 206; 256; 306; 406; 426; 466; Stoff 446) dazu ausgelegt ist, sich gemäß seines charakteristischen Profils der verzögerten Freisetzung mit kontrollierter Geschwindigkeit aufzulösen, nachdem das Implantat in das Subjekt implantiert wurde.

4. Implantat nach Anspruch 1, wobei jede der mindestens zwei Streben ein Paar verdrillter Drähte (152₁A, 152₁B, 152₂A, 152₁B; 202A, 202B, 202C; 252A, 252B, 252C; 302A, 302B, 302C; 402₁, 402₂; 462₁, 462₂) aufweist, die sich in Längsrichtung zwischen dem proximalen Ende und dem distalen Ende erstrecken.

5. Implantat nach Anspruch 1, wobei das Implantat (100; 150; 200; 250; 300; 400) dazu ausgelegt ist, in einer Hülle (114) in der komprimierten Auslegung eingehüllt zu werden, und / oder
wobei das Implantat (100; 150; 200, 250; 300; 400) zwischen der expandierten Auslegung und der komprimierten Auslegung weitere Komprimierungszustände hat.

6. Implantat nach Anspruch 1, wobei das Implantat (100; 150; 200; 250; 300; 400) zur Implantation in die Harnröhrenprothese des Subjekts und zum Aufbringen von Druck auf die umgebenden Gewebe der Harnröhrenprothese in der offenen Auslegung ausgelegt ist.

7. Implantat nach Anspruch 1, wobei der Anker (104; 154; 204; 254; 304) dazu ausgelegt ist, zu verhindern, dass das Implantat (100; 150; 200; 250; 300; 400) in Richtung Blase des Subjekts migriert.

8. Implantat nach Anspruch 1, wobei das Implantat (100; 150; 200; 250; 300; 400) aus dem Subjekt entfernbar ist.

9. Implantat für die Harnröhre eines Subjekts, wobei das Implantat (200; 250; 300; 400) umfasst:
drei formgeschlossene Drähte (202A, 202B, 202C; 252A, 252B, 252C; 302A, 302B, 302C), wobei jeder formgeschlossene Draht (202A, 202B, 202C; 252A, 252B, 252C; 302A, 302B, 302C) zwei Längsabschnitte aufweist, die sich zwischen einem proximalen Ende und einem distalen Ende des Implantats erstrecken, wobei jeder Längsabschnitt eines formgeschlossenen Drahts (202A, 202B, 202C; 252A, 252B, 252C; 302A, 302B, 302C) entlang seiner Länge an einen anderen Längsabschnitt eines anderen formgeschlossenen Drahts (202A, 202B, 202C; 252A, 252B, 252C; 302A, 302B, 302C) grenzt, wobei die formgeschlossenen Drähte (202A, 202B, 202C; 252A, 252B, 252C; 302A, 302B, 302C) dazu ausgelegt sind, Harnröhrengewebe an einer Struktur in der Harnröhre in Eingriff zu nehmen und zu expandieren; einen Anker (204; 254; 304), der einen formgeschlossenen Draht (204A, 204B; 254A, 254B; 304A, 304B) zum Verankern des Implantats an der Harnröhre aufweist, **gekennzeichnet durch** mindestens einen arzneimittelabgebenden Draht (206; 256; 306), der an mindestens einen der formgeschlossenen Drähte (202A, 202B, 202C; 252A, 252B, 252C; 302A, 302B, 302C) grenzt und sich in Längsrichtung entlang desselben erstreckt, wobei in den arzneimittelabgebenden Draht (206; 256; 306) ein Arzneimittel zur Freisetzung in der Harnröhre eingebettet ist.

10. Implantat nach Anspruch 9, wobei jeder von: den drei formgeschlossenen Drähten (202A, 202B, 202C; 252A, 252B, 252C; 302A, 302B, 302C), dem Anker (204; 254; 304) und dem mindestens einen arzneimittelabgebenden Draht (206; 256; 306) jeweils ein kontinuierlicher Draht ist, und / oder
ferner eine proximale Kappe (112; 162; 212; 262; 312) umfasst, die sich an dem proximalen Ende befindet, wobei die proximale Kappe (112; 162; 212; 262; 312) dazu ausgelegt ist, die Enden eines jeden der drei formgeschlossenen Drähte (202A, 202B, 202C; 252A, 252B, 252C; 302A, 302B, 302C), des Ankers (204; 254; 304) und des mindestens einen arzneimittelabgebenden Drahts (206; 256; 306) abzudecken.

11. Implantat nach Anspruch 9, wobei das Implantat (200; 250; 300; 400) zum Aufbringen von Druck auf die Gewebe der Harnröhrenprothese ausgelegt ist, und / oder
wobei in den mindestens einen arzneimittelabgebenden Draht (206; 256; 306) das Arzneimittel eingebettet ist, das ausgewählt wurde aus einer Liste bestehend aus: Sirolimus (Rapamycin) und Everolimus, und /oder wobei der mindestens eine arzneimittelabgebende Draht (206; 256; 306) spiralförmig um mindestens einen der drei formgeschlossenen Drähte (202A, 202B, 202C; 252A, 252B, 252C; 302A, 302B, 302C) gewunden ist, und / oder wobei der mindestens eine arzneimittelabgebende Draht (206; 256; 306) eine Vielzahl von arzneimittelabgebenden Drähten (466₁, 466₂, 466₃) aufweist, wobei in jeden arzneimittelabgebenden Draht ein anderes Arzneimittel eingebettet ist, und / oder
wobei der mindestens eine arzneimittelabgebende Draht aus mindestens einem einzelnen Strang von Material (Stoff 446) besteht, das dazu ausgelegt ist, das Arzneimittel zu absorbieren und abzusondern, und / oder
wobei der mindestens eine arzneimittelabgebende Draht (206; 256; 306; 406; 426; 466; Stoff 446) dazu ausgelegt ist, sich gemäß seinem charakteristischen Profil zur verzögerten Freisetzung mit kontrollierter Geschwindigkeit aufzulösen, nachdem das Implantat in das Subjekt implantiert wurde, und / oder
wobei der mindestens eine arzneimittelabgebende Draht mindestens zwei arzneimittelabgebende Drähte (206; 256; 306; 308) aufweist, wobei mindestens ein arzneimittelabgebender Draht (206; 256; 306) an mindestens einen der drei formgeschlossenen Drähte (202A, 202B, 202C; 252A, 252B, 252C; 302A, 302B, 302C) grenzt und mindestens ein anderer arzneimittelabgebender Draht (308) an den Anker (204; 254; 304) grenzt.

12. Implantat nach Anspruch 9, wobei das Implantat (200; 250; 300; 400) dazu ausgelegt ist, in einer Hülle (114) in der komprimierten Auslegung eingehüllt zu werden, und / oder
wobei das Implantat (200, 250; 300; 400) zwischen der expandierten Auslegung und der komprimierten Auslegung weitere Komprimierungszustände hat.

13. Implantat nach Anspruch 9, wobei das Implantat (200; 250; 300; 400) zur Implantation in die Harnröhrenprothese des Subjekts und zum Aufbringen von Druck auf die umgebenden Gewebe der Harnröhrenprothese in der offenen Auslegung ausgelegt ist.

14. Implantat nach Anspruch 9, wobei der Anker (204; 254; 304) dazu ausgelegt ist, zu verhindern, dass das Implantat (200; 250; 300; 400) in Richtung Blase des Subjekts migriert.

15. Implantat nach Anspruch 9, wobei das Implantat (200; 250; 300; 400) aus dem Subjekt entfernbar ist.

## Revendications

1. Implant (100 ; 150 ; 200 ; 250 ; 300 ; 400) pour l'urètre d'un sujet, l'implant (100 ; 150 ; 200 ; 250 ; 300 ; 400) comprenant :
au moins deux supports (102A ; 102B ; 152₁A, 152₁B, 152₂A, 152₁B ; 202A, 202B, 202C ; 252A, 252B, 252C ; 302A, 302B, §02C ; 402₁, 402₂ ; 462₁, 462₂) s'étendant longitudinalement entre une extrémité proximale et une extrémité distale dudit implant, chacun desdits supports (102A ; 102B ; 152₁A, 152₁B, 152₂A, 152₁B ; 202A, 202B, 202C ; 252A, 252B, 202C ; 302A, 302B, 302C ; 402₁, 402₂ ; 462₁, 462₂) étant flexible entre une configuration comprimée qui permet la mise en place dudit implant (100 ; 150 ; 200 ; 250 ; 300 ; 400) jusqu'à un rétrécissement dans ladite urètre, et une configuration dilatée qui facilite la dilatation dudit rétrécissement ; un ancrage (104 ; 154 ; 204 ; 254 ; 304) s'étendant vers l'extérieur à partir d'au moins l'une de ladite extrémité proximale et ladite extrémité distale, pour ancrer ledit implant (100 ; 150 ; 200 ; 250 ; 300 ; 400), **caractérisé en ce qu'**au moins un fil de distribution de médicament (106 ; 156 ; 206 ; 256 ; 306 ; 406 ; 426 ; 466 ; tissu 446), adjacent à au moins l'un desdits supports (102A ; 102B ; 152₁A, 152₁B, 152₂A, 152₁B ; 202A, 202B, 202C ; 252A, 252B, 252C ; 302A, 302B, 302C ; 402₁, 402₂ ; 462₁, 462₂), et s'étendant longitudinalement le long de ceux-ci, ledit fil de distribution de médicament (106 ; 156 ; 206 ; 256 ; 306 ; 406 ; 426 ; 466 ; tissu 446) est imprégné d'un médicament destiné à être libéré dans ladite urètre.

2. Implant selon la revendication 1, dans lequel chacun parmi lesdits au moins deux supports (102A ; 102B ; 152₁A, 152₁B, 152₂A, 152₁B ; 202A, 202B, 202C ; 252A, 252B, 252C ; 302A, 302B, 302C ; 402₁, 402₂ ; 462₁, 462₂), ledit ancrage (104 ; 154 ; 204 ; 254 ; 304) et ledit au moins un fil de distribution de médicament (106 ; 156 ; 206 ; 256 ; 306 ; 406 ; 426 ; 466 ; tissu 446) est respectivement un fil continu, et/ou
comprenant en outre un capuchon proximal (112 ; 162) situé à ladite extrémité proximale, ledit capuchon proximal (112 ; 162 ; 212 ; 262 ; 312) étant configuré pour recouvrir les extrémités de chacun desdits fils continus desdits au moins deux supports (102A ; 102B ; 152₁A, 152₁B, 152₂A, 152₁B ; 202A, 202B, 202C ; 252A, 252B, 252C ; 302A, 302B, 302C ; 402₁, 402₂ ; 462₁, 462₂), dudit ancrage (104 ; 154 ; 204 ; 254 ; 304) et dudit au moins un fil de distribution de médicament (106 ; 156 ; 206 ; 256 ; 306 ; 406 ; 426 ; 466 ; tissu 446).

3. Implant selon la revendication 1, dans lequel ledit implant (100 ; 150 ; 200 ; 250 ; 300 ; 400) est configuré pour appliquer une pression sur les tissus de ladite urètre prostatique, et/ou
ledit au moins un fil de distribution de médicament (106 ; 156 ; 206 ; 256 ; 306 ; 406 ; 426 ; 466 ; tissu 446) est imprégné dudit médicament choisi dans une liste constituée des médicaments suivants : sirolimus (rapamycine) ; et évérolimus, et/ou
ledit au moins un fil de distribution de médicament (106 ; 156 ; 206 ; 256 ; 306 ; 406 ; 426) est enroulé en spirale autour d'au moins l'un desdits supports, et/ou ledit au moins un fil de distribution de médicament (106 ; 156 ; 206 ; 256 ; 306 ; 406 ; 426 ; 466 ; tissu 446) inclut une pluralité de fils de distribution de médicament (106 ; 156 ; 206 ; 256 ; 306 ; 406 ; 426 ; 466₁, 466₂, 466₃), chaque fil de distribution de médicament étant imprégné d'un médicament différent, et/ou
ledit au moins un fil de distribution de médicament est constitué d'au moins un seul brin de matériau (tissu 446) qui est configuré pour absorber et également exsuder ledit médicament, et/ou
ledit au moins un fil de distribution de médicament (106 ; 156 ; 206 ; 256 ; 306 ; 406 ; 426 ; 466 ; tissu 446) est configuré pour se dissoudre à une vitesse contrôlée selon son profil caractéristique de libération prolongée, après que ledit implant a été implanté dans ledit sujet.

4. Implant selon la revendication 1, dans lequel chacun desdits au moins deux supports inclut une paire de fils torsadés (152₁A, 152₁B, 152₂A, 152₁B ; 202A, 202B, 202C ; 252A, 252B, 252C ; 302A, 302B, 302C ; 402₁, 402₂ ; 462₁, 462₂) s'étendant longitudinalement entre ladite extrémité proximale et ladite extrémité distale.

5. Implant selon la revendication 1, lequel implant (100 ; 150 ; 200 ; 250 ; 300 ; 400) est configuré pour être enveloppé dans une gaine (114) dans ladite configuration comprimée, et/ou
lequel implant (100 ; 150 ; 200 ; 250 ; 300 ; 400) a des états de compression intermédiaires entre ladite configuration dilatée et ladite configuration comprimée.

6. Implant selon la revendication 1, lequel implant (100 ; 150 ; 200 ; 250 ; 300 ; 400) est configuré pour être implanté dans l'urètre prostatique dudit sujet, et pour appliquer une pression sur les tissus entourant ladite urètre prostatique dans ladite configuration ouverte.

7. Implant selon la revendication 1, dans lequel ledit ancrage (104 ; 154 ; 204 ; 254 ; 304) est configuré pour empêcher ledit implant (100 ; 150 ; 200 ; 250 ; 300 ; 400) de migrer vers la vessie dudit sujet.

8. Implant selon la revendication 1, lequel implant (100 ; 150 ; 200 ; 250 ; 300 ; 400) peut être retiré du sujet.

9. Implant pour l'urètre d'un sujet, lequel implant (200 ; 250 ; 300 ; 400) comprend :
trois fils de forme fermée (202A, 202B, 202C ; 252A, 252B, 252C ; 302A, 302B, 302C), chaque fil de forme fermée (202A, 202B, 202C ; 252A, 252B, 252C ; 302A, 302B, 302C) incluant deux sections longitudinales s'étendant entre une extrémité proximale et une extrémité distale dudit implant, chaque section longitudinale d'un fil de forme fermée (202A, 202B, 202C ; 252A, 252B, 252C ; 302A, 302B, 302C) étant adjacente sur sa longueur à une autre desdites sections longitudinales d'un autre desdits fils à forme fermée (202A, 202B, 202C ; 252A, 252B, 252C ; 302A, 302B, 302C), lesdits fils à forme fermée (202A, 202B, 202C ; 252A, 252B, 252C ; 302A, 302B, 302C) étant configurés pour s'engager avec le tissu urétral, et le dilater, au niveau d'un rétrécissement dans ladite urètre ; un ancrage (204 ; 254 ; 304), incluant un fil de forme fermée (204A, 204B ; 254A, 254B ; 304A, 304B), pour l'ancrage dudit implant à ladite urètre, **caractérisé par** au moins un fil de distribution de médicament (206 ; 256 ; 306), adjacent à au moins l'un desdits fils de forme fermée (202A, 202B, 202C ; 252A, 252B, 252C ; 302A, 302B, 302C), et s'étendant longitudinalement le long de ceux-ci, ledit fil de distribution de médicament (206 ; 256 ; 306) étant imprégné d'un médicament destiné à être libéré dans ladite urètre.

10. Implant selon la revendication 9, dans lequel chacun : desdits trois fils de forme fermée (202A, 202B, 202C ; 252A, 252B, 252C ; 302A, 302B, 302C), dudit ancrage (204 ; 254 ; 304) et dudit au moins un fil de distribution de médicament (206 ; 256 ; 306) est respectivement un fil continu, et/ou
comprenant en outre un capuchon proximal (112 ; 162 ; 212 ; 262 ; 312) situé à ladite extrémité proximale, ledit capuchon proximal (112 ; 162 ; 212 ; 262 ; 312) étant configuré pour recouvrir les extrémités de chacun desdits trois fils de forme fermée (202A, 202B, 202C ; 252A, 252B, 252C ; 302A, 302B, 302C), dudit ancrage (204 ; 254 ; 304) et dudit au moins un fil de distribution de médicament (206 ; 256 ; 306).

11. Implant selon la revendication 9, lequel implant (200 ; 250 ; 300 ; 400) est configuré pour appliquer une pression sur les tissus de ladite urètre prostatique, et/ou
ledit au moins un fil de distribution de médicament (206 ; 256 ; 306) est imprégné dudit médicament choisi dans une liste constituée des médicaments suivants :
sirolimus (rapamycine) ; et évérolimus, et/ou ledit au moins un fil de distribution de médicament (206 ; 256 ; 306) est enroulé en spirale autour d'au moins l'un desdits trois fils à forme fermée (202A, 202B, 202C ; 252A, 252B, 252C ; 302A, 302B, 302C), et ledit au moins un fil de distribution de médicament (206 ; 256 ; 306) inclut une pluralité de au moins un fils de distribution de médicament (466₁, 466₂, 466₃), chaque au moins un fil de distribution de médicament étant imprégné d'un médicament différent, et/ou
ledit au moins un fil de distribution de médicament étant constitué d'au moins un seul brin de matériau (tissu 446) qui est configuré pour absorber et également exsuder ledit médicament, et/ou
ledit au moins un fil de distribution de médicament (206 ; 256 ; 306 ; 406 ; 426 ; 466 ; tissu 446) est configuré pour se dissoudre à une vitesse contrôlée selon son profil caractéristique de libération prolongée, après que ledit implant a été implanté dans ledit sujet, et/ou
ledit au moins un fil de distribution de médicament inclut au moins deux fils de distribution de médicament (206 ; 256 ; 306 ; 308), moyennant quoi au moins un fil de distribution de médicament (206 ; 256 ; 306) est adjacent à au moins l'un desdits fils à forme fermée (202A, 202B, 202C ; 252A, 252B, 252C ; 302A, 302B, 302C), et au moins un autre fil de distribution de médicament (308) est adjacent audit ancrage (204 ; 254 ; 304).

12. Implant selon la revendication 9, lequel implant (200 ; 250 ; 300 ; 400) est configuré pour être enveloppé dans une gaine (114) dans ladite configuration comprimée, et/ou
lequel implant (200 ; 250 ; 300 ; 400) a des états de compression intermédiaires entre ladite configuration dilatée et ladite configuration comprimée.

13. Implant selon la revendication 9, lequel implant (200 ; 250 ; 300 ; 400) est configuré pour être implanté dans l'urètre prostatique dudit sujet, et pour appliquer une pression sur les tissus entourant ladite urètre prostatique dans ladite configuration ouverte.

14. Implant selon la revendication 9, dans lequel ledit ancrage (204 ; 254 ; 304) est configuré pour empêcher ledit implant (200 ; 250 ; 300 ; 400) de migrer vers la vessie dudit sujet.

15. Implant selon la revendication 9, lequel implant (200 ; 250 ; 300 ; 400) peut être retiré du sujet.
